(19) Europäisches Patentamt
European Patent Office
Office européen des brevets

(11) **EP 4 775 165 A1**

(12) **EUROPEAN PATENT APPLICATION**
published in accordance with Art. 153(4) EPC

(43) Date of publication:
**15.07.2026 Bulletin 2026/29**

(21) Application number: **24852948.9**

(22) Date of filing: **16.08.2024**

(51) International Patent Classification (IPC):
*A61B 34/10* (2016.01)    *A61B 17/70* (2006.01)
*G06T 7/11* (2017.01)    *G06T 7/30* (2017.01)

(52) Cooperative Patent Classification (CPC):
**A61B 17/70; A61B 34/10; G06T 7/11; G06T 7/30**

(86) International application number:
**PCT/KR2024/012185**

(87) International publication number:
**WO 2025/053483 (13.03.2025 Gazette 2025/11)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 07.09.2023  KR 20230119273
11.01.2024  KR 20240004853
01.04.2024  PCT/KR2024/004196

(71) Applicant: **Curexo Inc.**
**Seoul 05814 (KR)**

(72) Inventors:
• **HWANG, Sung Teac**
**Seoul 03622 (KR)**
• **KANG, Hyun Jung**
**Seoul 01614 (KR)**
• **WOO, Dong Gi**
**Seoul 04722 (KR)**

(74) Representative: **Lavoix**
**Bayerstraße 83**
**80335 München (DE)**

(54) **IMAGE-BASED 3D AUTOMATIC SURGERY PLANNING METHOD AND SYSTEM**

(57) Provided are a three-dimensional (3D) image-based surgical planning method and system. The planning method includes: obtaining a first image and a second image from a spinal surgery site image; obtaining first and second baselines from the first image and the second image; and obtaining a third image from the spinal surgery site based on the first and second baselines, and determining a 3D screw path by setting proximal setting coordinates for determining a screw path in a central area of at least one of left and right third pedicles and distal setting coordinates in a center or a near center of the vertebra body region.

FIG. 1

## Description

### Technical Field

The present disclosure relates to an automatic surgical planning method and system in a surgical robot system, and particularly, to an automatic surgical planning method and system for generating a surgical planning path in a three-dimensional (3D) image space.

### Background Art

Spine surgical procedures, such as a pedicle screw fixation surgery using a surgical robot system, require three-dimensional (3D) images, for which an axial view is available. Therefore, rather than a two-dimensional (2D) image, a 3D image for which an axial view is available, for example, a computed tomography (CT) image, is used as an axial view image.

Generally, during intra-operation, a 2D image acquisition device, for example, a so-called C-arm, is used to verify in real time whether surgery is proceeding according to pre-surgery planning. To use the 2D image acquired as described above to navigate surgical tools as planned in advance, the 2D image acquired during intra-operation has to be registered with respect to a 3D image acquired before surgery. However, the process of registering a 2D image to a 3D image may result in delays in surgery time and registration errors due to incomplete registration.

On the other hand, a surgical plan based only on 3D images acquired before surgery without the help of 2D images during surgery cannot avoid the radiation exposure occurring when the 3D images, for example, CT images, are obtained, and the problem caused by the difference between patient's preoperative conditions and patient's intraoperative conditions.

However, if a surgical plan is established based on 2D X-ray images acquired during surgery, it is not necessary to register the images to images acquired before surgery, and the flexibility of changing the surgical plan according to necessity even during surgery may be expected. However, this process requires an operating surgeon to estimate a location in a 3D space in which the surgery is to be performed while viewing the 2D image. As a result, these surgical plans require a high level of surgical experience for the surgeon. Even in this case, a 2D image lacks image quality and image information according to 3D spatial image information, compared to a 3D CT image, and thus, it is difficult to expect a successful surgery unless the surgery is performed by an experienced surgeon.

Considering these aspects, it is highly desirable in many ways to develop an image-based surgical planning method and system that are based on an image system, rather than a subjective surgical plan dependent upon the experience of a surgeon.

<Prior Art Documents>

US8,335,553
CN107157579
US 7,787,932 B2
KR101264198 B1
US0659599 B2
US6226548 B1
US20070270866 A1
US20050192575 A1

### Disclosure of Invention

### Technical Problem

The present disclosure provides a method and system for automatically establishing a three-dimensional (3D) image-based surgical plan based on a plurality of images in different directions so that surgery may be performed based on the 3D image-based surgical plan.

The present disclosure provides a method of automatically establishing a 3D surgical plan by using three or more images in different directions obtained before surgery and/or during surgery and performing the surgery based on the automatically established 3D surgical plan and a system using the method.

**Solution to Problem**

A three-dimensional (3D) image-based surgical planning method according to the present disclosure includes:

obtaining, via one or more image acquisition devices, one or more surgery site images including image information in a plurality of directions including a first direction, a second direction, and a third direction which are different from one another, with respect to a spinal surgery site of a patient;
extracting, from the surgery site image via an image processor, a first image in the first direction and a second image in the second direction;
extracting, via an object segmentation portion, a first vertebra body region and a first pedicle region from the first image and a second vertebra body region and left and right second pedicle regions in the second vertebra body region from the second image;
setting, via a baseline setting portion, a first baseline passing through a first coordinate set in a central area of the first pedicle region and a second coordinate set in the first vertebra body region, and a second baseline passing through both of a third coordinate and fourth coordinates set in central areas of the left and right pedicle regions in the second image;
extracting, based on the first baseline and the second baseline via the image processor, a third image in the third direction from the surgery site image;
extracting, via the object segmentation portion, a third vertebra body region and left and right third pedicle regions from the third image; and
setting, via a path setting portion, a screw path passing through a central area of at least one of the left and right third pedicle regions and the third vertebra body region, while setting proximal setting coordinates in the second pedicle, and setting, on a third baseline passing through a foramen region between the left and right third pedicle regions and a central area of the vertebra body, distal setting coordinates through which a baseline of the screw path passes.

According to one or more embodiments,
the object extraction portion may extract the vertebra body region and the pedicle region by applying a model generated by machine learning.

According to one or more embodiments,
the first baseline may intersect a midline passing through a region between the first pedicle region and the first vertebra body region or cross the midline by an arbitrary angle, wherein the first baseline may be set to pass through the central area of the first pedicle region or to be parallel by being apart from the central area of the first pedicle region by a distance arbitrarily set.

According to one or more embodiments,
the central area may include a center and/or a near center of a corresponding vertebra body region and/or a pedicle region.

According to one or more embodiments,
the third image may be obtained from the surgery site image, based on a coordinate of the first baseline and a slope of the second baseline.

According to one or more embodiments,
the third image may be obtained from the surgery site image, based on coordinates of the first baseline of the first image and the second baseline of the second image, wherein the second baseline may commonly pass through the central area of each of the left and right pedicles in the second image.

According to one or more embodiments,
the second baseline may be set to be parallel with an arbitrary straight line connecting a midpoint between a center of a pedicle region at one side of the left and right pedicle regions, and a coordinate of a corner at one side of the third image adjacent to the center of the pedicle region with a midpoint between a center of a pedicle region at the other side of the left and right pedicle regions and a coordinate of a corner at the other side of the third image adjacent to the center of the pedicle region.

According to one or more embodiments,

the object segmentation portion may extract the foramen region between the left and right third pedicle regions from the third image, and
the path setting portion
may set, in the third image, the third baseline commonly passing through coordinates of a center or a near center of each of the third vertebra body region and the foramen region, and
may set the distal setting coordinates on a point at which the third baseline crosses a boundary line of a vertebra body region most distanced apart from the foramen region or a position near to the point, or on a crossing point at which the third baseline and the baseline of the screw path cross each other outside the vertebra body.

According to one or more embodiments,
the path setting portion may set the baseline of the screw path on which the screw path is arranged to pass through the distal setting coordinates.

According to one or more embodiments,
a screw start point with respect to a pedicle and a target point in a vertebra body may be set on the first baseline, and a distance between the screw start point and the target point may be set as a length of a screw used in surgery.

According to one or more embodiments,
the first pedicle region may have an inner edge facing the first vertebra body region and an outer edge at an opposite side to the inner edge, the start point may be positioned on the outer edge of the first pedicle region or to be adjacent to the outer edge, and the target point may be positioned in the first vertebra body region.

According to one or more embodiments,
the start point may be arranged on an edge at one side of the second pedicle region toward an outside of the second vertebra body region or to be adjacent to the edge in the second vertebra body region, and the target point may be arranged on an edge at another side opposite to the one side or to be adjacent to the edge in the second vertebra body region.

According to one or more embodiments,
the surgery site image including the image information in the plurality of directions may be obtained by the 3D image acquisition device.

A 3D image-based surgical planning system according to the present disclosure includes:

one or more image acquisition devices obtaining one or more surgery site images having image elements in a plurality of directions with respect to a spinal surgery site;

an image processor configured to process images related to a surgery, including extraction of a first image in a first direction, a second image in a second direction different from the first direction, and a third image in a third direction different from the first direction and the second direction from the one or more surgery site images;

an object segmentation portion extracting a first vertebra body region and a first pedicle region corresponding to a vertebra body and a pedicle from the first image, a second vertebra body region and a second pedicle region in the second vertebra body region from the second image, and a third vertebra body region and a third pedicle region from the third image;

a baseline setting portion setting a first baseline passing through a first coordinate set in a central area of the first pedicle region and a second coordinate set in the first vertebra body region and a second baseline commonly passing through a third coordinate and fourth coordinates set in central areas of the left and right pedicle regions in the second image; and

a screw path setting portion setting a screw path passing through a central area of at least one of the left and right third pedicle regions and the third vertebra body region while setting proximal setting coordinates in the second pedicle and setting, on a third baseline passing through a foramen region between the left and right third pedicle regions and a central area of the vertebra body, distal setting coordinates through which a baseline of the screw path passes.

According to one or more embodiments, in the 3D image-based surgical planning system, the baseline setting portion may configure the first baseline to perpendicularly cross a midline passing through a region between the first pedicle region and the first vertebra body region.

According to one or more embodiments, in the 3D image-based surgical planning system, the image processor may obtain the third image from the surgery site image, based on a coordinate of the first baseline and a slope of the second baseline.

According to one or more embodiments, in the 3D image-based surgical planning system, the image processor may obtain the third image from the surgery site image, based on coordinates of the first baseline of the first image and the second baseline of the second image, wherein the second baseline may commonly pass through the central area of each of the left and right pedicles in the second image.

According to one or more embodiments, in the 3D image-based surgical planning system, the second baseline may be set to be parallel with an arbitrary straight line connecting a midpoint between a center of a pedicle region at one side of the left and right pedicle regions, and a coordinate of a corner at one side of the third image adjacent to the center of the pedicle region with a midpoint between a center of a pedicle region at the other side and a coordinate of a corner at the other side of the third image adjacent to the center of the pedicle region.

According to one or more embodiments, in the 3D image-based surgical planning system, the object segmentation portion may extract the foramen region between the left and right third pedicle regions from the third image, and

the path setting portion
may set, in the third image, the third baseline commonly passing through coordinates of a center or a near center of each of the third vertebra body region and the foramen region, and

may set the distal setting coordinates on a point at which the third baseline crosses a boundary line of a vertebra body region most distanced apart from the foramen region or a position near to the point, or on a crossing point at which the third baseline and the baseline of the screw path cross each other outside the vertebra body.

According to one or more embodiments, in the 3D image-based surgical planning system, the path setting portion may set the baseline of the screw path on which the screw path is arranged to pass through the distal setting coordinates.

According to one or more embodiments, in the 3D image-based surgical planning system, a screw start point with respect to a pedicle and a target point in a vertebra body may be set on the first baseline, and a distance between the screw start point and the target point (or a terminating point) may be set as a length of a screw used in surgery.

**Advantageous Effects of Invention**

The present disclosure provides a method and system for automatically establishing a three-dimensional (3D) surgical plan by using a plurality of pieces of image information. According to the present disclosure, the limit of a surgical method, for example, a previous problem of a surgical plan, that is, limits in the surgical result based on the experience of an operating surgeon, may be resolved, to efficiently establish and execute a surgical plan. According to the present disclosure, the surgical plan may be automatically generated, and thus, the overall operation time may be reduced, and operation results may be maintained at a high level.

**Description of Drawings**

FIG. 1 is a flowchart of time-sequential processing operations of a three-dimensional (3D) image-based surgical planning method according to one or more embodiments of the present disclosure.

FIG. 2 schematically shows definition of 3D coordinates with respect to a virtual 3D surgical space of a first image and a second image, according to one or more embodiments of the present disclosure.

FIG. 3 shows an output image in which a vertebra body and a pedicle are extracted, segmented, or separated from a lateral-lateral (LL) image by applying a first image segmentation model, according to one or more embodiments of the present disclosure.

FIG. 4 shows an output image in which a vertebra body and a pedicle are segmented from an anterior-posterior (AP) image by applying a second image segmentation model, according to one or more embodiments of the present disclosure.

FIG. 5 schematically shows a generation process of a deep learning model applied in one or more embodiments of the present disclosure.

FIG. 6 shows an image in which a vertebra body and a pedicle are labelled in a plurality of first images for learning, according to one or more embodiments of the present disclosure.

FIG. 7 shows an image in which a vertebra body and a pedicle are labelled in a plurality of second images for learning, according to one or more embodiments of the present disclosure.

FIG. 8 shows an image in which a vertebra body, a pedicle, and a foramen are labelled in a plurality of third images for learning, according to one or more embodiments of the present disclosure.

FIG. 9 shows results of labelling segmented targets in a first image and a second image, according to one or more embodiments of the present disclosure.

FIG. 10 illustrates a firstly selected target L1, according to one or more embodiments of the present disclosure.

FIG. 11 illustrates a vertebra body region (A) and a pedicle region (B) set in a first image, according to one or more embodiments of the present disclosure.

FIG. 12 is a y-z planar graph showing vertex coordinates of a vertebra body region (A) and a pedicle region (B) set in a first image and a midline between these regions, according to one or more embodiments of the present disclosure.

FIG. 13 is a y-z planar graph showing a temporary straight line obtained by rotating the midline of FIG. 11 by 90 degrees, according to one or more embodiments of the present disclosure.

FIG. 14A is a y-z planar graph showing an initial first baseline D1 arranged in parallel with a temporary straight line D', according to one or more embodiments of the present disclosure.

FIG. 14B is a y-z planar graph showing a modified first baseline D1a substituting for the first baseline D1 of FIG. 14A and arranged in parallel therewith, according to one or more embodiments of the present disclosure.

FIG. 15A is a y-z planar graph showing a coordinate of a target point T or a terminating point of a surgical screw, which is set on the initial first baseline of FIG. 14A, according to one or more embodiments of the present disclosure.

FIG. 15B is a y-z planar graph showing a target point T or a terminating point of a surgical screw, which is set on the modified first baseline of FIG. 14B, according to one or more embodiments of the present disclosure.

FIG. 16 is an image showing a state in which a second vertebra body region (a) and second pedicle regions (b) and (b') are extracted or separated through a first image segmentation model, according to one or more embodiments of the present disclosure.

FIG. 17 is an x-z planar graph showing, by dotted lines, oval-shaped second pedicle regions (b) and (b') within a second vertebra body region (a) with vertices (a1), (a2), (a3), and (a4), according to one or more embodiments of the present disclosure.

FIG. 18 illustrates a state in which a first baseline and a second baseline are set in obtained first and second images, according to one or more embodiments.

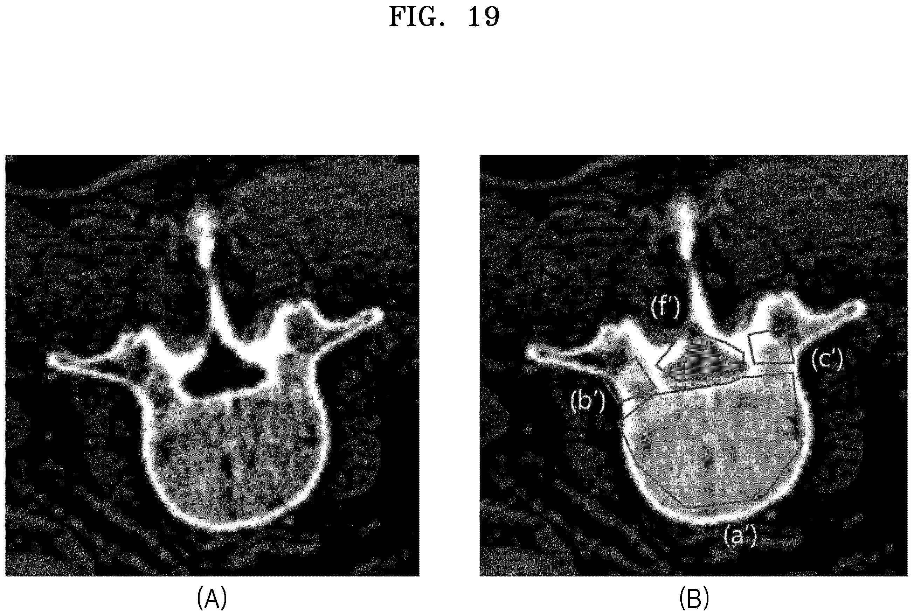

(A) of FIG. 19 illustrates a third image obtained along a slice path in an axial direction, according to one or more embodiments, and (B) of FIG. 19 illustrates a vertebra body region, a pedicle region, and a foramen region as the region of interest (ROI) extracted from the third image.

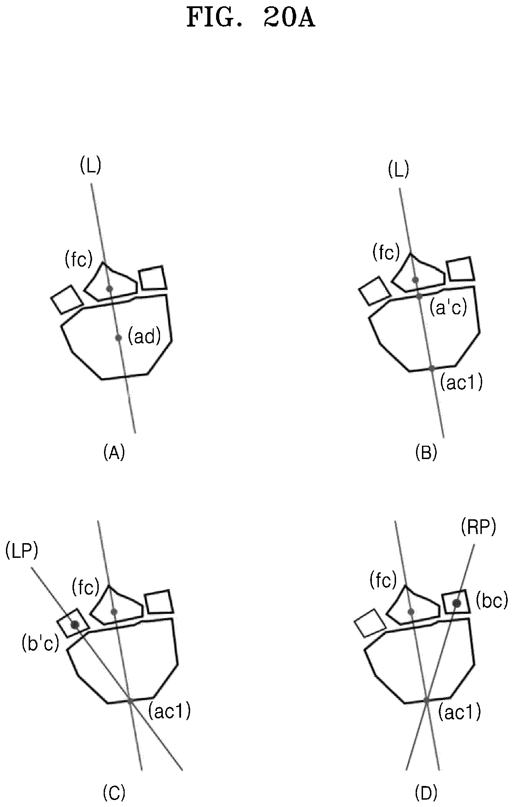

FIG. 20A illustrates an example of a process of setting a screw path baseline with respect to the ROI of a third image, according to one or more embodiments.

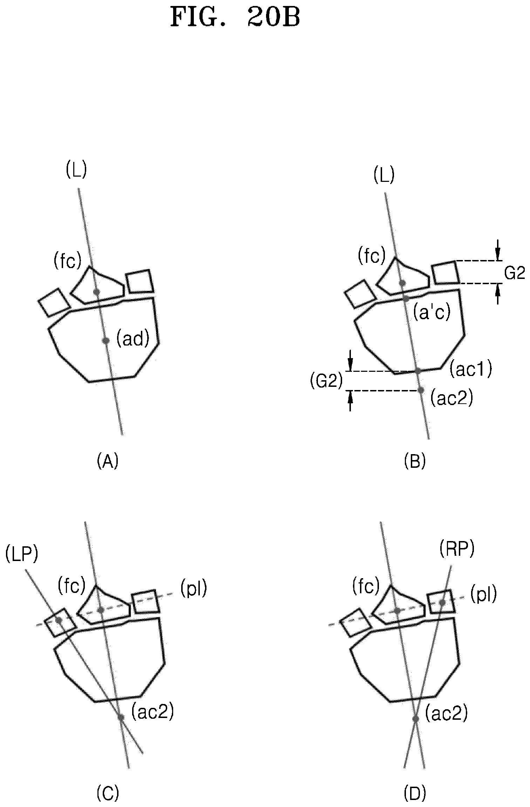

FIG. 20B illustrates another example of a process of setting an alternative screw path baseline with respect to the ROI of a third image, according to one or more embodiments.

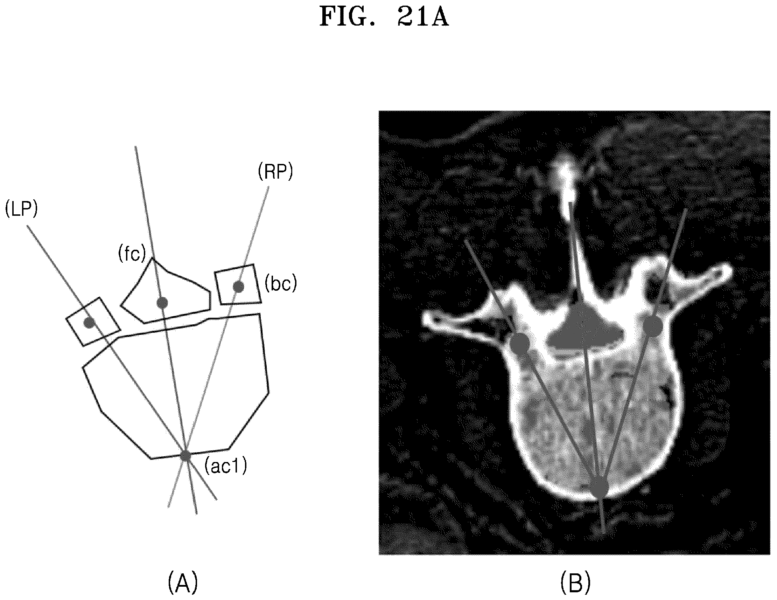

FIG. 21A illustrates the ROI and a third image, in which a screw path baseline with respect to the ROI of the third image is set, according to an embodiment of FIG. 20A.

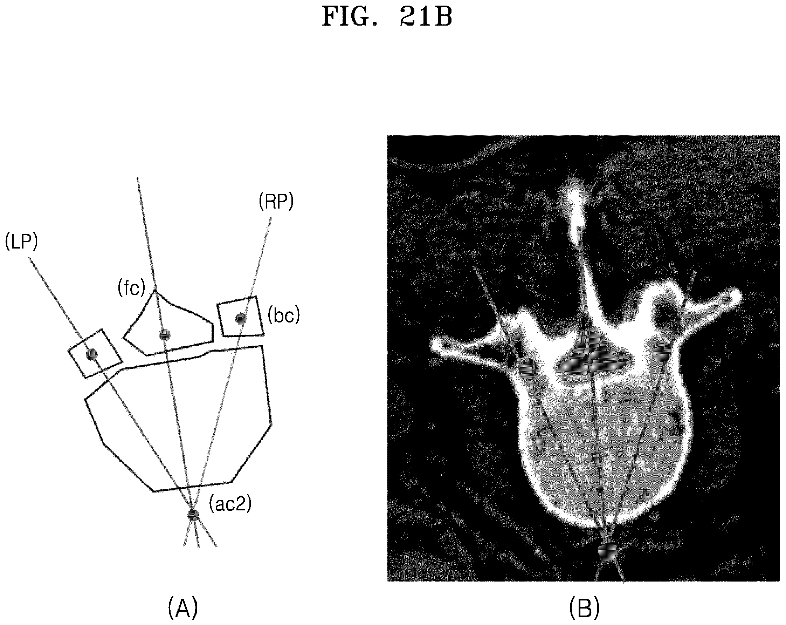

FIG. 21B illustrates the ROI and a third image, in which a screw path baseline with respect to the ROI of the third image is set, according to an embodiment of FIG. 20B.

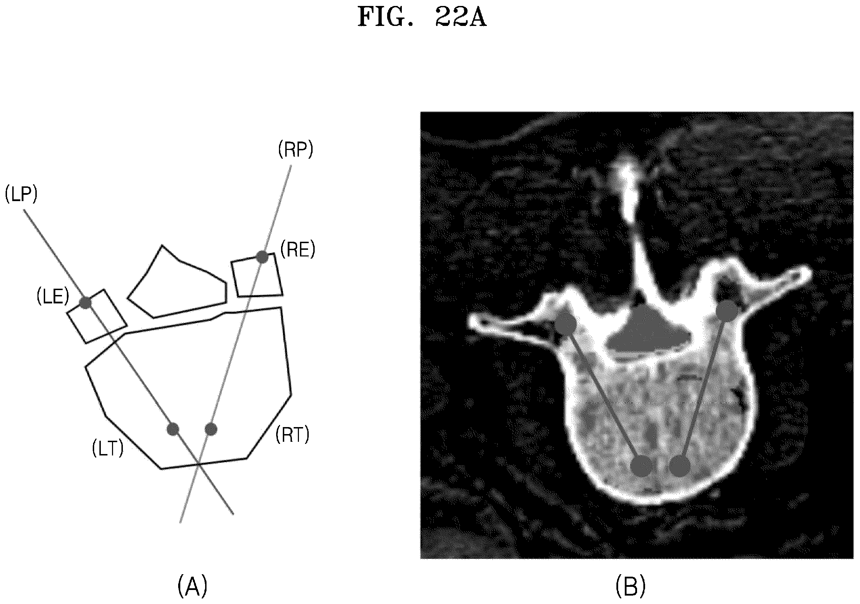

FIG. 22A illustrates a state in which an entry point and a target point (or a terminating point) of a screw path of the total length are set on the screw path baseline of FIG. 21A, according to an embodiment of FIG. 20A.

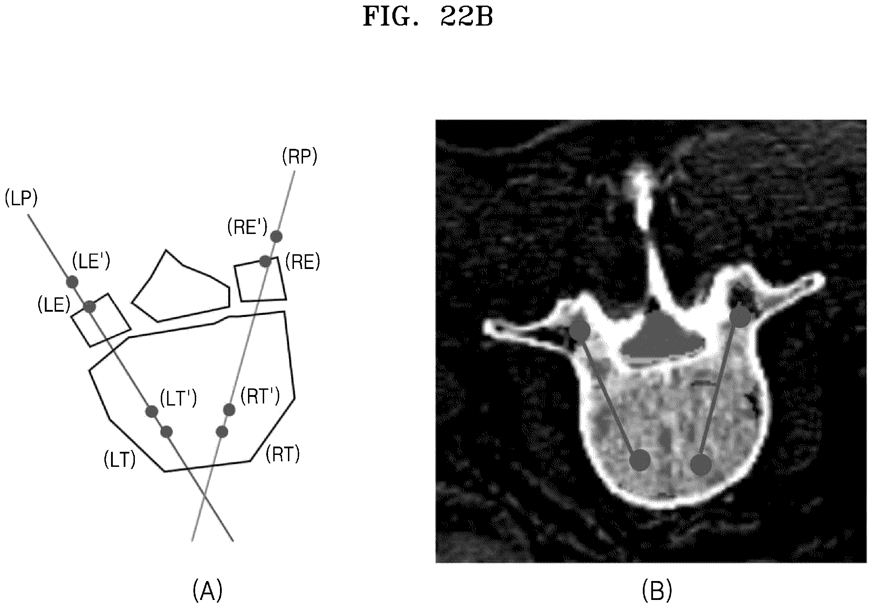

FIG. 22B illustrates a state in which an entry point and a target point (or a terminating point) of a screw path of the total length are set on the screw path baseline of FIG. 21B, according to an embodiment of FIG. 20B.

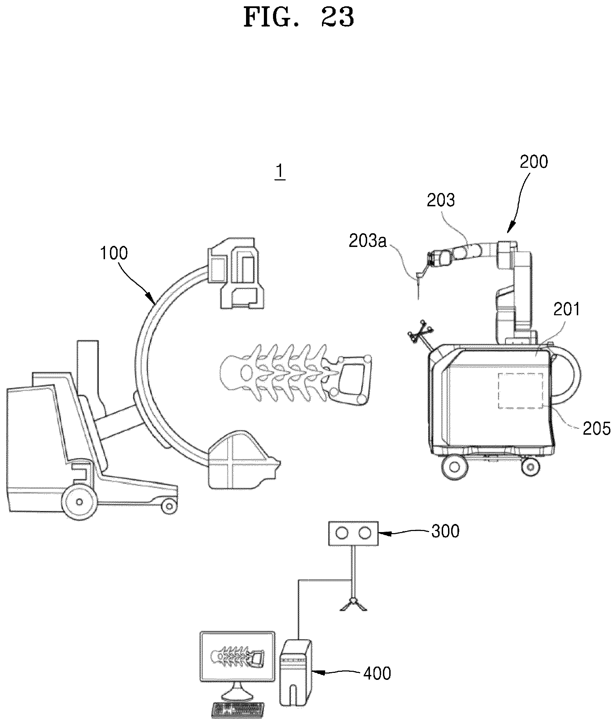

FIG. 23 shows a schematic structure of a surgical robot system according to one or more embodiments of the present disclosure.

**Mode for the Invention**

Hereinafter, preferred embodiments of the present inventive concept will be described in detail with reference to the accompanying drawings. However, the embodiments of the present inventive concept may be modified into various other forms, and the scope of the present inventive concept should not be construed as being limited to the embodiments described in detail below. It is preferable that the embodiments of the present inventive concept be interpreted as being provided to more completely explain the present inventive concept to those with average knowledge in the art. Identical symbols refer to identical elements throughout. Furthermore, various elements and areas in the drawings are schematically drawn. Thus, the inventive concept is not limited by the relative sizes or distances shown in the accompanying drawings.

The terms, such as "first," "second," etc., may be used to describe various components, but the components shall not be limited by the terms. These terms are used only for distinguishing one element from another element. For example, without deviating from the scope of the claims of the present disclosure, a first element may be referred to as a second element, and in contrast, the second element may be referred to as the first element.

Terms used in the present application are used only for describing particular embodiments of the present disclosure and are not intended to limit the present disclosure. A singular expression may include a plural expression, unless an apparently different meaning is indicated in the context. With respect to the present application, it will be further understood that the expressions "comprises" and "comprising" used herein specify the presence of stated features, integers, steps, operations, members, components, and/or groups thereof, but do not preclude the presence or addition of one or more other features, integers, operations, members, components, and/or groups thereof.

Unless defined otherwise, all of the terms used herein, including technical terms and scientific terms, have the same meaning as the meaning commonly understood by one of ordinary skill in the art. Also, the terms commonly used and having the meanings as defined in dictionaries shall be understood to have consistent meanings with the corresponding terms in the context of relevant arts, and unless explicitly defined so herein, the meanings of the terms shall not be understood to be excessively formal.

When a certain embodiment may be implemented differently, a specific process order may be performed differently from the described order. For example, two consecutively described processes may be performed substantially at the same time or performed in an order opposite to the described order.

Hereinafter, a three-dimensional (3D) image-based surgical planning method and system will be described in detail according to one or more embodiments.

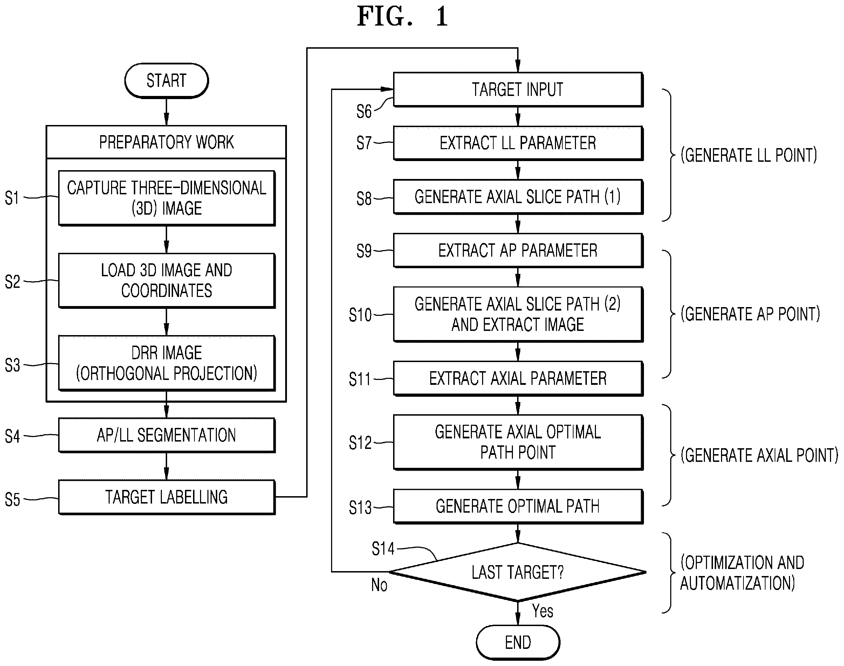

FIG. 1 is a time-sequential flowchart of a 3D image-based surgical planning method according to the present disclosure.

The process of FIG. 1 includes operations S6 to S14 that are repeatedly performed on a plurality of vertebra bodies which are targets, and when there is only one vertebra body, a surgical plan for one vertebra is established by performing the process once.

According to one or more embodiments described hereinafter, descriptions are given based on a 3D image by a 3D image acquisition device. However, it is obvious that embodiments of the present disclosure are not limited by the 3D image by the 3D image acquisition device. The present disclosure may include all devices which may manufacture an image by one or more image acquisition devices which may capture an image of image information in three different directions, in particular, assembled or registered images in three-directions.

FIG. 1 is described below according to each operation.

In general, the method according to the present disclosure includes a preparatory work process, an LL point generation process, an AP point generation process, an axial (hereinafter, often AX)-direction point generation process, and an optimization and automation process.

<Operation S1>

Operation S1 is an operation in which a plurality of images are captured with respect to a patient's affected area in different directions. This operation may use a 3D image by a 3D image acquisition device according to the present embodiment.

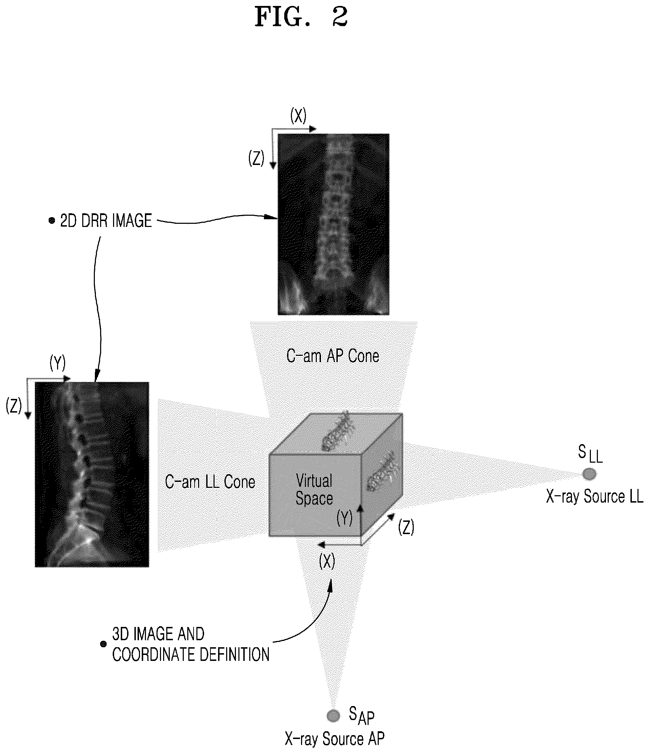

FIG. 2 schematically shows definition of 3D coordinates with respect to a virtual 3D surgical space of a first image and a second image, according to one or more embodiments of the present disclosure.

<Operation S2>

Operation S2 is an operation in which a 3D image having axes in X, Y, and Z directions is loaded to an image processor to be processed. In this operation, the 3D image and coordinate information are loaded.

<Operation S3>

Operation S3 is an operation in which the loaded 3D image is processed in two directions to obtain a first image and a second image as digitally reconstructed radiograph (DRR) images. In this operation, for example, a lateral-lateral (LL) image is obtained as the first image, and for example, an anterior-posterior (AP) image is obtained as the second image. These images may be obtained by orthogonal projection in a first direction and a second direction with respect to the 3D image.

In the LL image, that is, the first image, a left and right direction (belly-back) indicates a Y axis, and a top and bottom direction (head-foot) indicates a Z axis. Also, in the AP image, that is, the second image, a top and bottom direction (head-foot) indicates a Z axis, and a left and right direction (left arm-right arm) indicates an X axis, and thus, the first image, for example, the LL image, is parallel with a Y-Z plane, and the second image, for example, the AP image is parallel with an X-Z plane.

<Operation S4>

In operation S4, the extraction or segmentation or separation of a vertebra body and a pedicle as target objects from the first and second images obtained in the process described above is performed.

The segmentation of the object may be performed by applying an LL segmentation model and an AP segmentation model trained by deep learning.

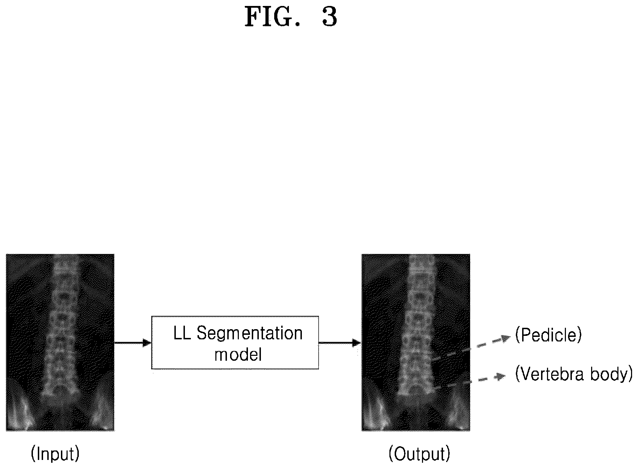

FIG. 3 shows an output image in which a vertebra body and a pedicle are segmented from an LL image by applying a first image segmentation model.

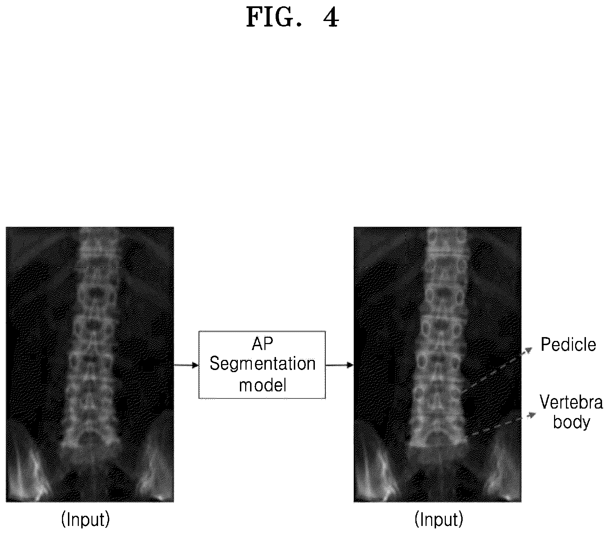

FIG. 4 shows an output image in which a vertebra body and a pedicle are segmented from an AP image by applying a second image segmentation model.

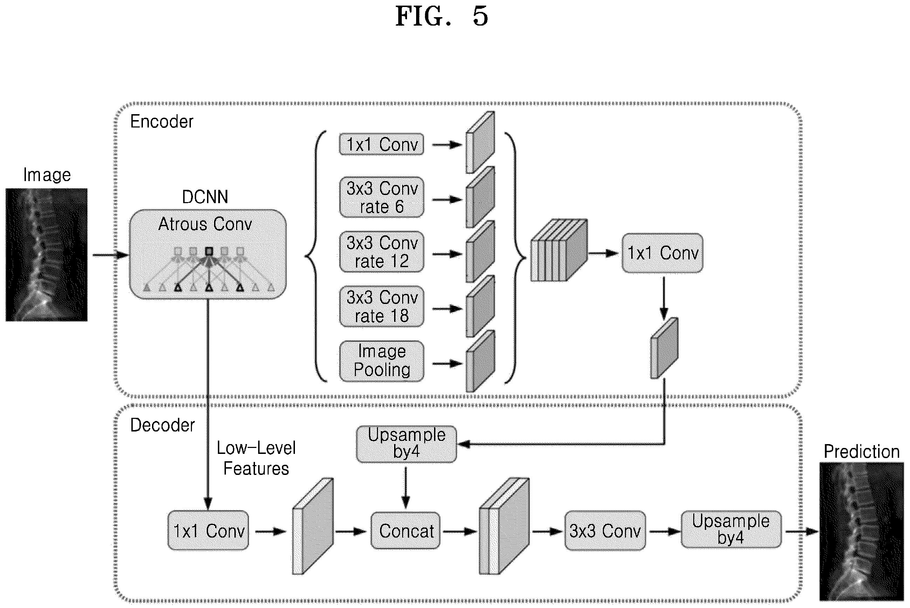

As illustrated in FIG. 5, the segmentation models described above may be based on a DeepLabv3+ model, and it is obvious that other models may also be applied according to other embodiments of the present disclosure.

The DeepLavb3+ model is one of the deep learning models used in the field of computer vision and is used to perform extraction or segmentation tasks. This model is based on a deep convolutional neural network (CNN) architecture and particularly, has an encoder and decoder structure so as to be suitable for image segmentation.

Training of the model includes the same process as general deep learning training. For the training of the model as described above, various vertebra-related image resources collected for training are required. Training images include a first image for training captured in a first direction and a second image for training captured in a second direction, and labelling at the pixel level is performed on the first image for training and the second image for training and then a learning process is performed, as illustrated in FIGS. 4 and 5, so that the targeted first and second image segmentation models may be acquired.

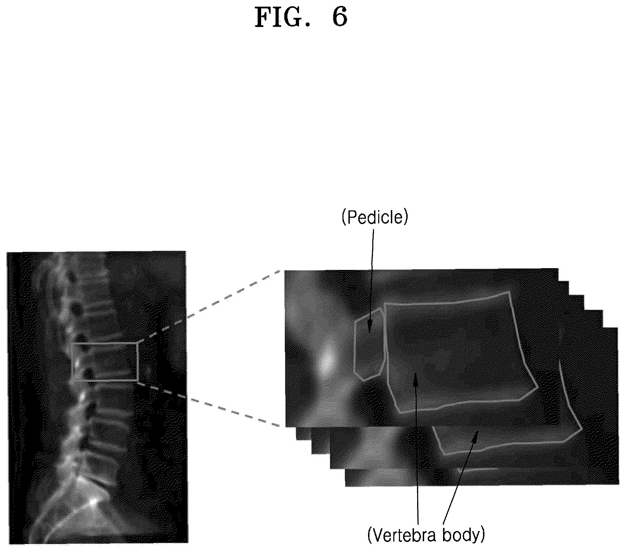

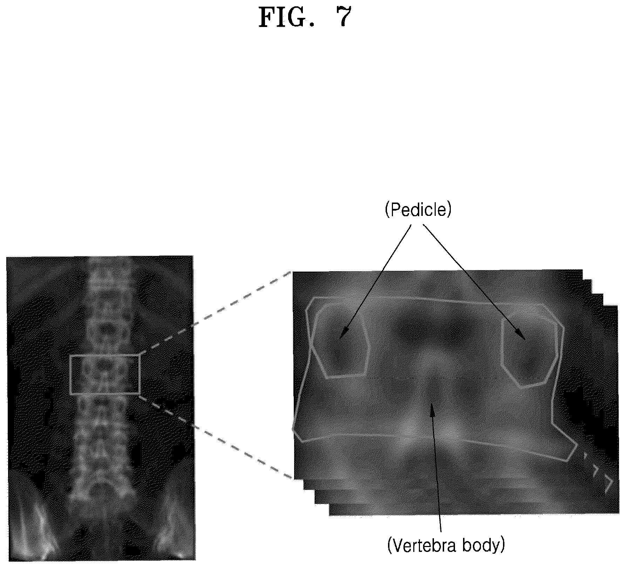

FIGS. 6 and 7 show images in which a vertebra body and a pedicle are labelled in a plurality of first images for training and a plurality of second images for training, respectively.

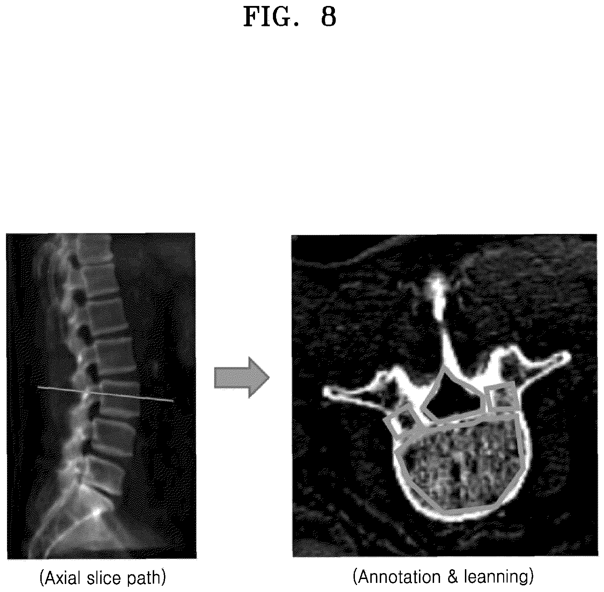

FIG. 8 shows an image in which a vertebra body, a pedicle, and a foramen are labelled in a plurality of third images for learning, according to one or more embodiments of the present disclosure.

<Operation S5>

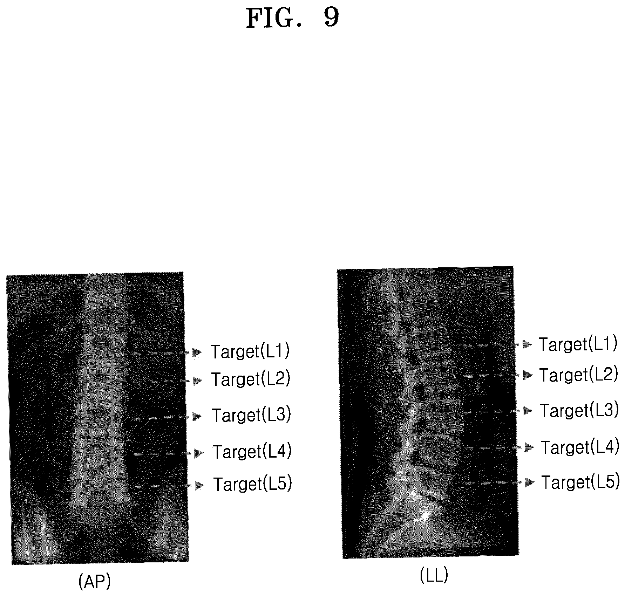

In this operation, labelling is performed on the targets segmented in the previous operation. FIG. 9 shows results of labelling the segmented targets in the AP image and the LL image.

As shown in FIG. 9, the segmented targets are labelled as L1, L2, L3, L3, L4, and L5 sequentially from the top, and the same label is assigned to the same vertebra body.

<Operation S6>

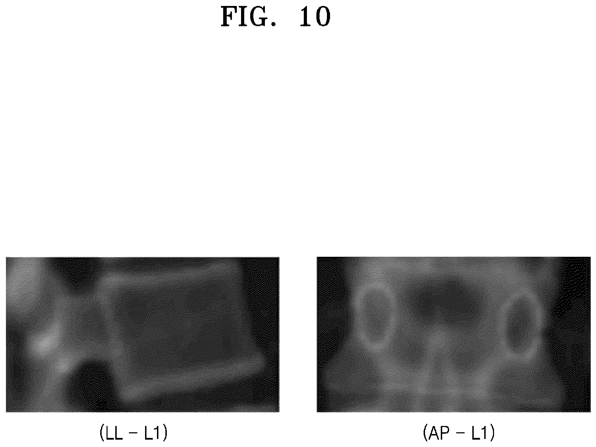

As illustrated in FIG. 10, in this operation, before a process of extracting an LL point, one target is selected from among the plurality of targets. For example, the target L1 may be selected first and may undergo a subsequent process.

<Operations S7 and S8>

The extraction of an LL parameter with respect to the selected labelled target is performed, and through this, a first baseline on an LL image plane is determined. This baseline corresponds to one axis of an image plane in a third direction and is a first axial slice path 1 to extract the third image described above. When planning the first baseline, the first baseline has to be set not to deviate from pedicle and vertebra body regions. Here, if the slope of only one of the vertebra body region and the pedicle region is reflected, the path may deviate from the remaining region, and in order to prevent path planning failure due to detection errors occurring during object segmentation, the first baseline may be set as the average slope of the slopes of the two regions.

To this end, first, the region of interest (ROI) with respect to the pedicle and vertebra body is set.

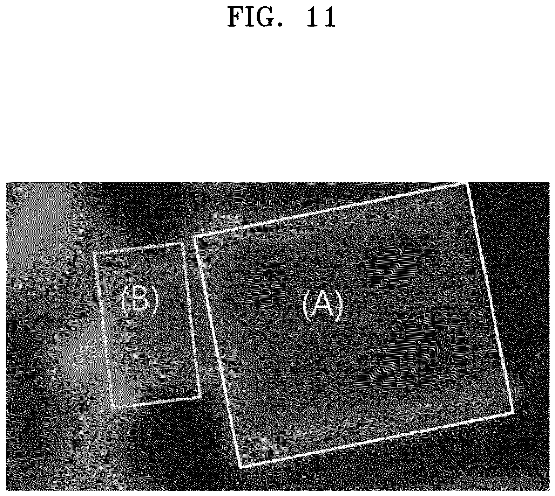

In FIG. 11, (A) is the vertebra body region, and (B) is the pedicle region. The ROI can be obtained by the first image segmentation model described above.

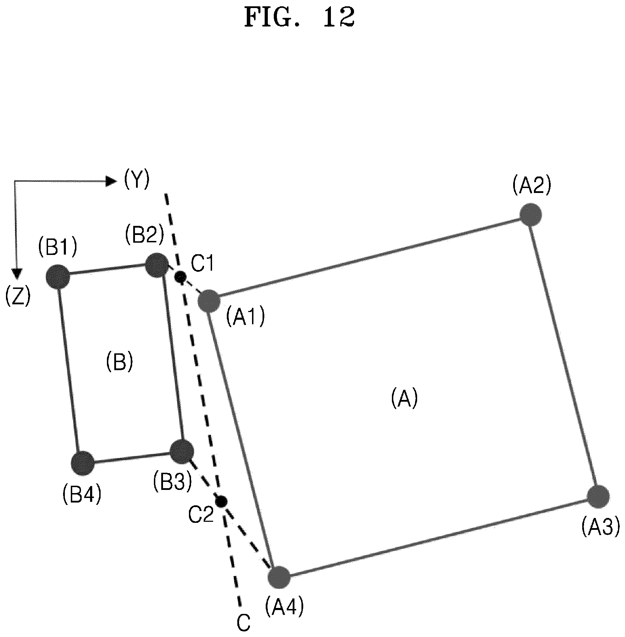

As illustrated in FIG. 12, the vertebra body region (A) is a quadrangular region with four vertices (A1), (A2), (A3) and (A4), the pedicle region (B) is a quadrangular region with four vertices (B1), (B2), (B3) and (B4), and the vertebra body region (A) and the pedicle region (B) are adjacent to each other. A midline C passes between adjacent sides of the two adjacent regions.

The midline C passes through the exact center between two adjacent sides (B2)-(B3) and (A1)-(A4) of the both regions (A) and (B), and intersection points C1 and C2 are located on the midline C. The intersection point C1 is located in the middle of a line segment connecting the vertices (B2) and (A1) of the both regions, and the intersection point C2 is located in the middle of a line segment connecting the vertices (B3) and (A4). Therefore, a y coordinate (C1.y) of C1 is (A1.y+B2.y) / 2, and a z coordinate (C1.z) of C1 is (A1.z+B2.z) / 2. Also, a y coordinate (C2.y) of C2 is (A4.y+B3.y) / 2, and a z coordinate (C2.z) of C2 is (A4.z+B3.z) / 2.

Thus, the midline C has a planar coordinate of an LL image, that is, an average slope Ca of the slopes of both regions on a y-z plane. The midline C serves as a reference for setting the first baseline on an LL image plane, and the coordinates C1(y, z) and C2(y, z) of the intersection points C1 and C2 in the y-z planar coordinate system and a slope Ca of the midline C are expressed by the equations below.

$$C1(y, z) = \left(\frac{A1.y+B2.y}{2}, \frac{A1.z+B2.z}{2}\right)$$

$$C2(y, z) = \left(\frac{A4.y+B3.y}{2}, \frac{A4.z+B3.z}{2}\right)$$

$$Ca = \frac{C2.z - C1.z}{C2.y - C1.y}$$

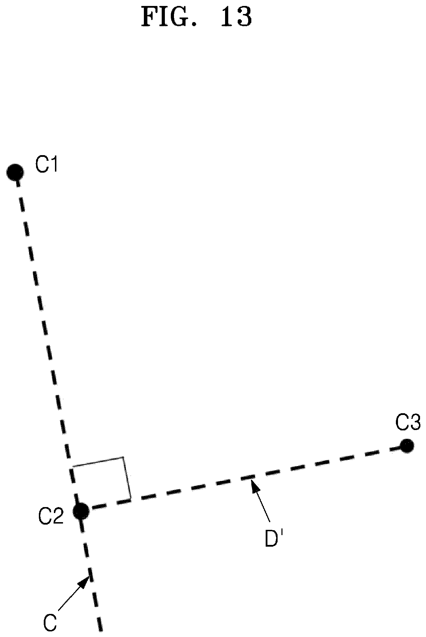

FIG. 13 shows a temporary point C3 in an intermediate process of setting the first baseline from the midline C between the pedicle region (B) and the vertebra body region (A) on the y-z plane.

The coordinate of the temporary point C3 corresponds to the coordinate obtained by rotating the intersection point C1 by 90 degrees with respect to the intersection point C2 on the midline C. That is, when the coordinate of C1 on the y-z planar coordinate system is (y1, z1), the coordinate of C3 becomes (z1, -y1). As a result, a temporary straight line D' connecting the intersection point C2 with the temporary point C3 is a normal line perpendicular to the midline C, and the straight line equation of the normal line is D':Z = Da(Y - C2.y) + C2.z.

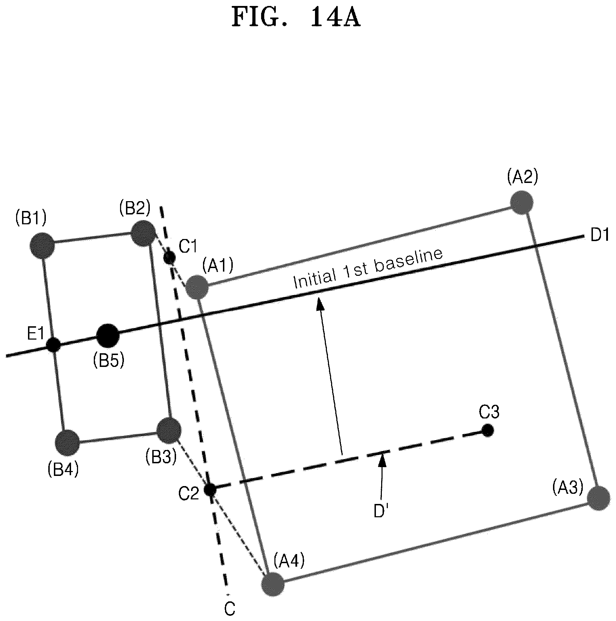

FIG. 14A illustrates an initial first baseline D1 arranged in parallel with the temporary straight line D'. The initial first baseline D1 crosses the midline C obliquely by a certain degree or intersects the midline C and is parallel with the temporary straight line D'. The temporary straight line D' is shifted in parallel by a certain distance so as to pass through a central area, that is, a center (B5) or the near center, of the pedicle region (B) having the four vertices such as (B1), (B2), (B3), and (B4), in order to determine the first initial baseline D1 on the y-z plane.

A y coordinate (B5.y) and a z coordinate (B5.z) of (B5) are expressed as follows.

$$B5.y = (B1.y + B2.y + B3.y + B4.y) / 4$$

$$B5.z = (B1.z + B2.z + B3.z + B4.z) / 4$$

The straight line equation of the first baseline D1 having a slope Da is expressed as follows.

$$D1:Z = Da (Y - C2.y) + C2.z$$

$$Da = (C3.z - C2.z) / (C3.y - C2.y)$$

The first baseline D1 expressed by the straight line equation D1:z as described above generates an intersection point E1 as the first baseline D1 passes through outer sides (BS) and (B1)-(B4) of the pedicle, and the intersection point E1 becomes a start point or an entry point to determine the length of a surgical screw which is to be described below.

The outer side BS of the pedicle is expressed by the equation as below.

$$BS: Z = Ba(Y - B1.y) + B1.z$$

$$Ba = (B4.z - B1.z) / (B4.y - B1.y)$$

Next, the coordinates of the start point located on the initial first baseline D1 and a target point, which is a terminating point, where the tip of the surgical screw reaches, are calculated.

With respect to coordinates (E.y, E.z) of the start point E located on the outer side of the pedicle, it is possible to calculate the y coordinate (E.y) and the z coordinate (E.z) of the start point by using the straight line equation D1:Z of the first baseline and a linear simultaneous equation applying the straight line equation BS:Z of the outer side (BS) of the pedicle.

$$D1:Z = Da (Y - C2.y) + C2.z$$

$$BS: Z = Ba(Y - B1.y) + B1.z$$

First, Y and Z of the two same straight lines are the same at (E.y) and (E.z), and thus, the two lines are calculated as below.

$$D1a (Y - C2.y) + C2.z = Ba(Y - B1.y) + B1.z$$

The value of (E.y) may be obtained by gathering the sections with respect to Y in each side and separating Y from each other in the equation above.

$$E.y = Y = (B1.z - C2.z + Ba * B1.y - Da * C2.y) / (Da - Ba)$$

By substituting (E.y) obtained in the equation above into the straight line equation D1:Z of the baseline D1, the coordinate (E.z) of the start point in the z direction may be obtained.

$$E.z = Z = Da (E.y - C2.y) + C2.z$$

The calculation process above is one of various calculation methods to obtain the coordinates of the start point E, which may be calculated by other methods, and it is obvious that a specific calculation method does not limit the technical scope of the present disclosure.

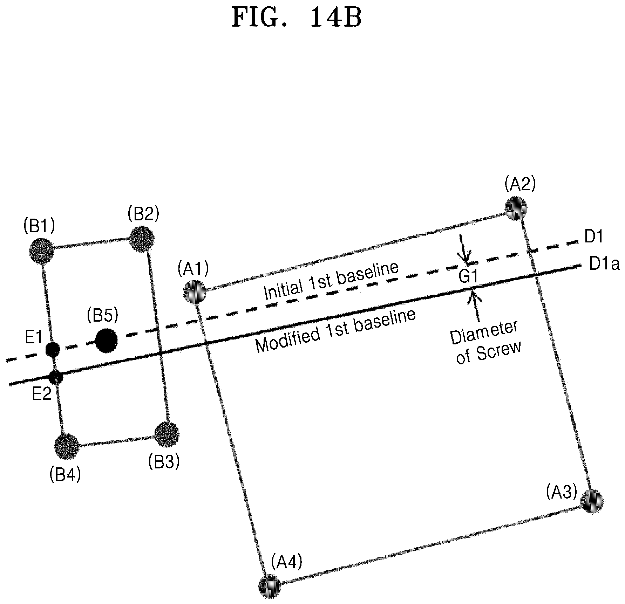

FIG. 14B shows the setting of a modified first baseline D1a obtained based on the initial first baseline D1, according to another embodiment of the present disclosure. The modified first baseline D1a substitutes for the initial first baseline D1 obtained in FIG. 14A.

The modified first baseline D1a is obtained by shifting, in parallel, the initial first baseline D1 toward a central direction of the vertebra body. Also, a parallel shift distance G1 may correspond to a diameter or a radius of a screw to be used in surgery, and according to another embodiment, may be greater or less than the diameter of the screw.

As described above, the shifting of the initial baseline in parallel toward the inside of the vertebra body is to prevent the screw from being exposed to the outside of the vertebra body due to the size of the screw, and depending on the patient's state, when the pedicle is not greatly offset with respect to the vertebra body, the initial first baseline may be applied as a screw path baseline. However, according to certain reasons or when, intrinsically, the pedicle is greatly offset with respect to the vertebra body, the modified first baseline may be used as the baseline of the screw path.

That is, according to an embodiment of the present disclosure, any one of the baselines D1 and D1a is selectively applied as the baseline for setting a final first screw path, and the first baseline described hereinafter is D1 or D1a.

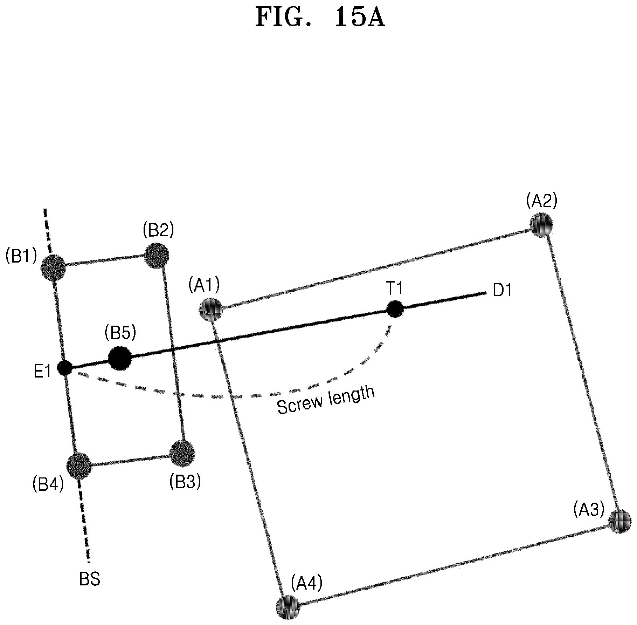

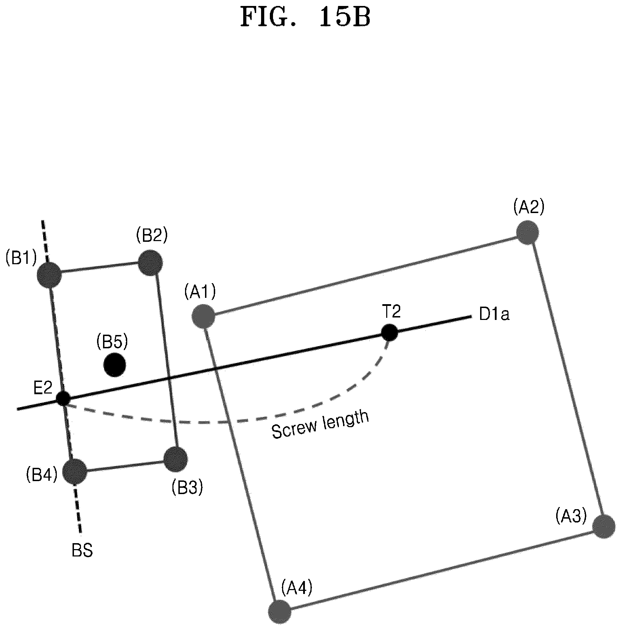

FIGS. 15A and 15B show the setting of entry points E1 and E2 and target points T1 and T2 of a surgical screw on the initial first baseline D1 and the modified first baseline, respectively.

First, referring to FIG. 15A, on a y-z plane, the target point T, which is a terminating point, at which a tip of the surgical screw is located, is set on the initial first baseline D1.

The coordinate of the target point T is determined by the total length Ls of the screw to be used. According to an embodiment of the present disclosure, the total length Ls of the screw is set not to exceed the length of the side (A1)-(A2) of the vertebra body region in a direction in which the surgical screw advances, and the diameter Ds of the screw is set not to be greater than the length of the outer side (B1)-(B4) where the entry point of the screw is located.

$$Ls = \sqrt{(A1.y - A2.y)^2 + (A1.z - A2.z)^2}$$

$$Ds = \sqrt{(B1.y - B4.y)^2 + (B1.z - B4.z)^2}$$

Here, coordinates T(y, z) of the target point are expressed by the equations below.

$$T.y = E.y + Ls * Cos(arctan(Da))$$

$$T.z = E.z + Ls * Sin(arctan(Da))$$

The length of a line segment connecting the coordinates of the entry point and the coordinates of the target point, obtained by the process described above, corresponds to the length of the screw to be used in surgery.

Referring to FIG. 15B, the entry point E2 and the target point T2 of the surgical screw are set on the modified first screw path D1a.

The coordinates of the start point E2 and the target point T2 on the modified first baseline D1a are set, based on the calculation of the equation described above. As illustrated in FIG. 14B, the modified first baseline D1a passes beyond the center B5 of the pedicle in the pedicle to pass through a near area.

<Operations S9 and S10>

In this operation, a second baseline, which is included in a pair with the first baseline in the y-z plane, that is, an axial slice path 2, is obtained.

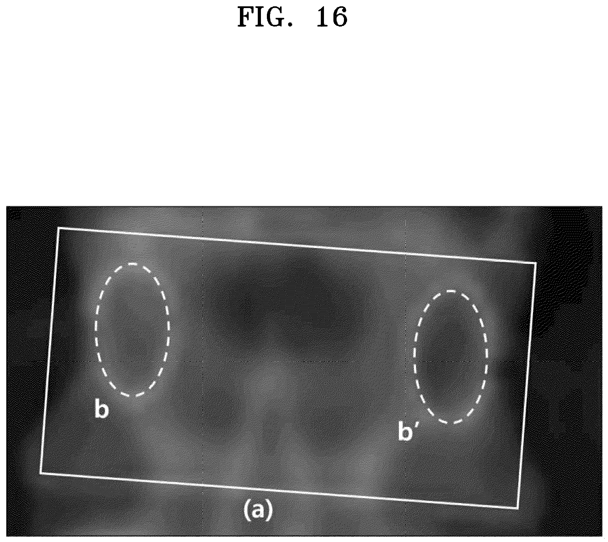

FIG. 16 shows an image in which a second vertebra body region (a) and second pedicle regions (b) and (b') are extracted or separated by using the first image segmentation model.

As illustrated in FIG. 16, the ROI (a) of the vertebra body and the left and right pedicles (b) and (b') may be extracted through a segmentation model with respect to the second image, that is, the AP image.

An important point in the setting of the second baseline in the AP image is to obtain an image of a slice section in which the left/right pedicles are shown to be the greatest when the third image described below, that is, an AX image, is obtained. To this end, the degree of rotation of the vertebra body has to be reflected, so that the pedicle region of the maximum size may be obtained in the AX image.

Thus, an average slope value is obtained to reduce a segmentation error between the slope of the ROI (a) of the vertebra body and the both left and right pedicle regions (b) and (b').

The average slope increases or decreases according to the shape of an arrangement of the left and right pedicles, and a temporary second baseline d' indicating the average slope is obtained as follows.

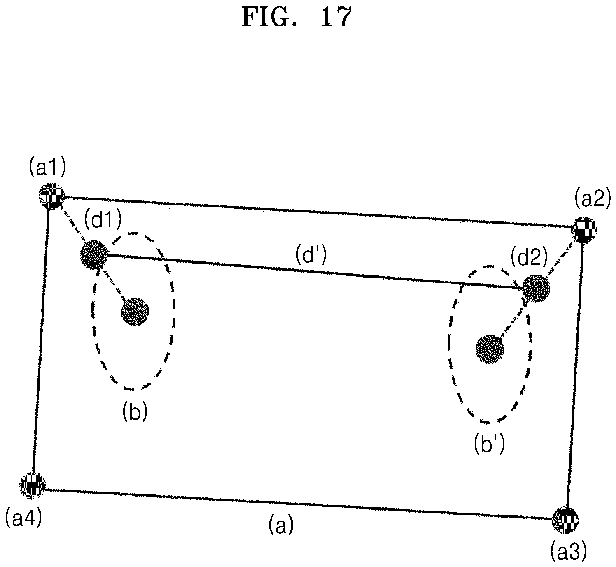

FIG. 17 is an x-z planar graph showing, by dotted lines, oval-shaped second pedicle regions (b) and (b') within a second vertebra body region (a) with vertices (a1), (a2), (a3), and (a4).

As illustrated in FIG. 17, first, a central point (d1) between a center (b5) of the left pedicle (b) and a corner (a1) of the vertebra body region most adjacent thereto and a central point (d2) between a center (c5) of the right pedicle (b') and a corner (a2) of the vertebra body region most adjacent thereto are obtained, and the two central points (d1) and (d2) are connected to acquire the temporary second baseline (d') having the average slope.

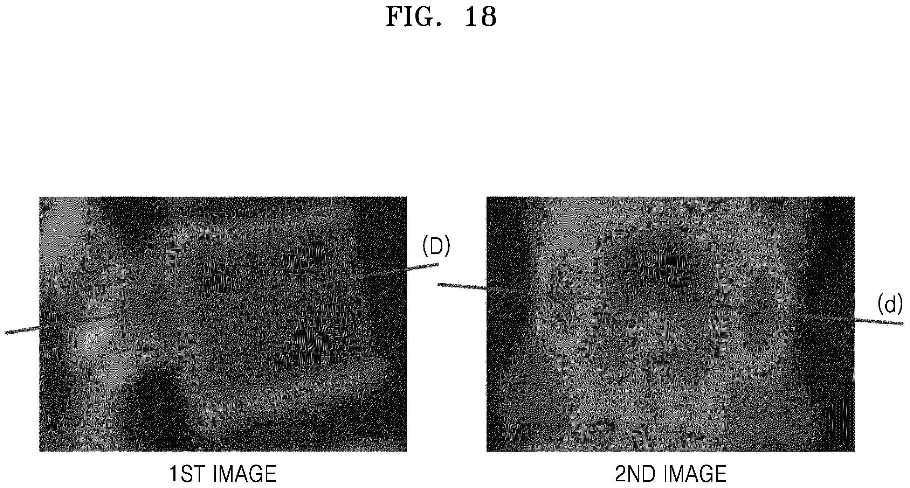

FIG. 18 shows the setting of a first baseline and a second baseline in the first image and the second image.

First, referring to FIG. 18, when the third image is obtained in the AX plane of the 3D image, by applying a Y-Z position of the first baseline D1 or D1a in the first image obtained in the process described above and the slope of the temporary baseline (d') in the X-Z plane in the second image, a final second baseline (d) passing through the center of the both pedicles on average is obtained as in the second image on the right side of FIG. 18. Thus, by this process, the third image in an axial direction, that is, the AX image, may be obtained from a slice plane according to the first baseline and the second baseline as illustrated in (A) of FIG. 19.

Here, the temporary second baseline (d') illustrated in FIG. 17 is illustrated across upper portions of the both pedicles (b) and (b') and is illustrated in FIG. 18 to pass through both central areas, that is, the near centers, of the both pedicles (b) and (b'). In FIG. 18, the second baseline (d) indicates the final second baseline when the slope of the second baseline in the X-Z plane is applied to the Y-Z position of the first baseline. That is, the final second baseline is obtained as a result of shifting the temporary second baseline (d') in parallel toward the average center of the both pedicles (b) and (b').

<Operation S11>

(A) of FIG. 19 illustrates the third image described above, and (B) of FIG. 19 illustrates a vertebra body region, a pedicle region, and a foramen region as the ROI extracted from the third image.

This operation is an operation in which an AX parameter with respect to an AX image of (A) of FIG. 19, obtained as described above, is extracted.

The extraction of the regions is performed by an object segmentation portion, and the object segmentation may be performed on the AX image by using an AX segmentation model, which is trained by deep learning using samples labelled with respect to the vertebra body, the pedicle, and the foramen, as illustrated in FIG. 18, like the LL segmentation model and the AP segmentation model trained by deep learning as described above.

(B) of FIG. 19 shows the vertebra body region (a), the left and right pedicle regions (b) and (b'), and the foramen region (f) between the left and right pedicles, which are extracted via the segmentation on the AX image.

FIGS. 20A and 20B illustrate a process of setting a screw path baseline with respect to the ROI of the third image, according to other embodiments.

First, the embodiment of FIG. 20A is described.

(A) to (D) of FIG. 20A show the process of setting the screw path baseline on which a screw path is placed in an AX image corresponding to a plane for surgical screw insertion or arrangement.

(A) After a central point (fc) is set in the central area of the foramen region and a central point (ad) is set in each central area of the vertebra body region, a third baseline (L) passing through the two points (fc) and (ad) is set.

(B) Intersection points (a'c) and (ac) between the third baseline and inner and outer boundary lines of the vertebra body region are set. The intersection point (ac) becomes a distal setting coordinate through which the screw path baseline passes. Also, the distance between (a'c) and (ac) indicates the width of the vertebra body in a direction of the third baseline and may be applied to determine the total length of a screw.

(C) A first screw path baseline (LP) passing through the intersection point (ac) and a center (b'c) of the pedicle at one side (the left side in the drawing), that is, a proximal setting coordinate, is set.

(D) A second screw path baseline (RP) passing through the intersection point (ac) and a center (bc) of the pedicle on the other side (the right side in the drawing), that is, a distal setting coordinate, is set.

The first and second screw path baselines (LP) and (RP) set as described above are axial lines on which the surgical screw is mounted.

The first and second screw path baselines (LP) and (RP) described above are set in slightly different directions through the method described below.

The embodiment of FIG. 20B is described.

In FIG. 20B, like FIG. 20A, (A) to (D) show the process of setting the screw path baseline on which a screw path is placed in an AX image corresponding to a plane for surgical screw insertion or arrangement.

(A) After a central point (fc) is set in the central area of the foramen region and a central point (ad) is set in each central area of the vertebra body region, a third baseline (L) passing through the two points (fc) and (ad) is set.

(B) Intersection points (a'c) and (ac1) between the third baseline and inner and outer boundary lines of the vertebra body region are set. The intersection point (ac1) becomes a distal setting coordinate through which the screw path baseline passes. Here, a modified intersection point (ac1') apart from the vertebra body from the intersection point (ac1) by a distal distance, that is, the distal setting coordinate, is set on the screw path baseline (L). The modified intersection point (ac1') is distanced apart from the intersection point (ac1) thereabove by an arbitrary distance G2.

The arbitrary distance G2 is the average thickness or height G2 of the pedicle, and according to another embodiment, may have a positive value less than or greater than the average thickness or height G2 of the pedicle.

Also, the distance between (a'c) and (ac1) indicates the width of the vertebra body in a direction of the third baseline and may be applied to determine the total length of a screw.

(C) A first screw path baseline (LP) passing through the modified intersection point (ac2) and a center (b'c) of the pedicle at one side (the left side in the drawing) is set.

(D) A second screw path baseline (RP) passing through the intersection point (ac2) and a center (bc) of the pedicle on the other side (the right side in the drawing) is set.

The first and second screw path baselines (LP) and (RP) set as above are modified axial lines on which a surgical screw is set, and an angle between the first and second screw path baselines (LP) and (RP) is reduced compared to an angle between the first and second screw path baselines (LP) and (RP) obtained according to the process of FIG. 20. Thus, both front end portions of the surgical screw arranged on the both baselines (LP) and (RP) do not meet each other in the vertebra body region.

(A) of each of FIGS. 22A and 22B is according to the embodiment of FIG. 20A or 20B and shows the first and second screw path baselines set according to the process described above, and (B) of each of FIGS. 22A and 22B illustrates a state in which the baseline is mapped to (overlaid on) an AX image.

As illustrated in (B) of FIG. 22A according to the embodiment of FIG. 20A, a start point or an entry point LE or RE of a surgical screw is formed where the first or second screw path baseline crosses an outer boundary line of each of both pedicles. The entry point is set on a proximal setting coordinate through which the screw path baseline passes.

Also, as illustrated in (B) of FIG. 22B according to the embodiment of FIG. 20B, the first and second screw path baselines pass through the centers (b'c, bc) and the modified intersection point (ac2) of each pedicle and the entry point of the screw is set on an outer surface of the pedicle.

<Operations S12 and S13>

Operations S12 and S13 are operations in which screw target points RT and LT apart from the start points LE and RE which are the entry points of the surgical screw, are finally set, and a final surgical plan is confirmed.

The entry point is located on the proximal setting coordinate on which the screw enters into the pedicle.

In this operation, the total length Ls of the screw obtained in the process of setting the first baseline described above or the width of the vertebra body in a direction of the first baseline may be applied to determine the length of the screw. Alternatively, the length of the screw may be determined by taking into account the width (the distance between (a'c) and (ac)) of the vertebra body in a direction of the third baseline together. Also, according to necessity, the length of the screw may be adjusted by an operating surgeon by taking into account the result above. Also, it is necessary to set the diameter of the screw to be less than the average diameter of the pedicle or a diameter of a smaller pedicle of the left and right pedicles. This aspect may also be modified by the surgeon.

(A) of each of FIGS. 22A and 22B according to the embodiment of FIG. 20A or 20B illustrates a screw path in which the total length Ls of the screw is applied to the ROI region, and (B) of each of FIGS. 22A and 22B illustrates a state in which the screw path is determined in the third image.

The screw path has 3D coordinates, which are recognized by a surgical system during surgery, so that the surgery is directly performed.

FIGS. 22A and 22B illustrate a state in which an entry point and a target point (or a terminating point) of a screw path having the total length are set on the screw path baseline of FIGS. 22A and 22B, according to one or more embodiments.

Referring to FIG. 22A according to the embodiment of FIG. 20A, the entry points LE and RE and the target points LT and RT, which are positioned on the surface of the pedicle, are formed on the first and second screw path baselines LP and RP, and the distance between the entry point LE and the target point LT on each baseline corresponds to the total length of the screw.

Referring to FIG. 22B according to the embodiment of FIG. 20B, modified entry points LE' and RE' are set. The modified entry points LE' and RE' are apart from the entry points LE and RE on the surface of the pedicle on the corresponding baseline by an arbitrary distance. This distance is configured by taking into account a gap due to the skin and/or muscle tissues of a patient, while the screw does not directly touch a rear end (the head) of the screw held by a surgical drill when the screw is fixed to the pedicle during actual surgery. Thus, the target points in the vertebra body are shifted to modified positions LT' and RT' from the original positions LT and LP. The positions of the entry point and the target point may be flexibly modified and changed on the screw path baseline determined in the process described above, according to a state of a surgery site of a patient and the determination of an operating surgeon.

The processes described above are performed with respect to one target. However, when there are multiple targets, that is, when there are a plurality of vertebral bodies, which are the surgical objects, and when the last target has not yet been reached in operation S14, the processes may return to operation S6 and operation S6 to operation S14 are repeatedly performed before the processes are ended.

As described above, according to an embodiment of the present disclosure, an automatic 3D surgical planning method and system based on an image are provided. According to the present disclosure, it is possible to perform a safe and definite surgery, without largely depending on the level of skill or the experience of an operating surgeon.

The embodiment according to the present disclosure may be realized by a computer program medium storing software for executing, on a computer, the method of automatically establishing the surgical plan described above. Also, the present disclosure may be realized by an image processor, an object segmentation portion, a baseline setting portion, a path setting portion, etc. in which a processor, a memory, a display, etc. are used.

Additionally, the present disclosure may also be realized by an image-based surgical robot system.

Referring to FIG. 23, a surgical robot system 1 according to one or more embodiments of the present disclosure includes an image acquisition device 100 as a main image acquisition device. In addition, the robot system 1 is formed by including a surgical robot 200, a position sensor 300, and a navigation system 400, and the surgical robot 200 is formed by including a main body 201, a robot arm 203 including an end effector 203a, and a robot controller 205.

According to an embodiment of the present disclosure, except for the image acquisition device described above, the image processor, the object segmentation portion, the baseline generator, and the path determination portion may be functionally included in the devices described above. Also, the image processor, the object separation portion, the baseline setting portion, the path setting portion, etc. may be realized by surgical planning software. In particular, the object separation portion may perform extraction or separation of a vertebra body and a pedicle from a first image, a second image, and a third image by applying an LL segmentation model, an AP segmentation model, and an AX segmentation model trained by deep learning.

According to the disclosure, the robot arm 203 is controlled according to a surgical plan determined during surgery and control software. The navigation system 400 may display, through a display, information about a surgical tool or a surgical plan about an implant on an image acquired during surgery or display a real time position of the surgical tool or the implant on the image or, in some cases, a 3D image acquired before surgery, thereby assisting the doctor in performing the surgery. To this end, the display that allows the doctor to view, with the naked eye, during surgery, the real time position of the surgical tool, etc. in comparison with the surgical plan and the surgical status, may be connected to the navigation system 400.

Although various embodiments of the present disclosure have been described in detail above, one of ordinary skill in the art may implement the present disclosure by modifying the embodiments of the present disclosure in various ways without deviating from the concept and the range of the present disclosure defined in the accompanying claims. Therefore, changes in the embodiments of the present disclosure shall not deviate from the description of the present disclosure.

## Claims

1.   A three-dimensional (3D) image-based surgical planning method comprising:

obtaining, via one or more image acquisition devices, one or more surgery site images comprising image information in a plurality of directions including a first direction, a second direction, and a third direction which are different from one another, with respect to a spinal surgery site of a patient;
extracting, from the surgery site image via an image processor, a first image in the first direction and a second image in the second direction;
extracting, via an object segmentation portion, a first vertebra body region and a first pedicle region from the first image and a second vertebra body region and left and right second pedicle regions in the second vertebra body region from the second image;
setting, via a baseline setting portion, a first baseline passing through a first coordinate set in a central area of the first pedicle region and a second coordinate set in the first vertebra body region and a second baseline passing through both of a third coordinate and fourth coordinates set in central areas of the left and right pedicle regions in the second image;

extracting, based on the first baseline and the second baseline via the image processor, a third image in the third direction from the surgery site image;

extracting, via the object segmentation portion, a third vertebra body region and left and right third pedicle regions from the third image; and

setting, via a path setting portion, a screw path passing through a central area of at least one of the left and right third pedicle regions and the third vertebra body region, while setting proximal setting coordinates in the second pedicle, and setting, on a third baseline passing through a foramen region between the left and right third pedicle regions and a central area of the vertebra body, distal setting coordinates through which a baseline of the screw path passes.

2. The 3D image-based surgical planning method of claim 1, wherein the object extraction portion extracts the vertebra body region and the pedicle region by applying a model generated by machine learning.

3. The 3D image-based surgical planning method of claim 1, wherein the first baseline intersects a midline passing through a region between the first pedicle region and the first vertebra body region or crosses the midline by an arbitrary angle, wherein the first baseline is set to pass through the central area of the first pedicle region or to be parallel by being apart from the central area of the first pedicle region by a distance arbitrarily set.

4. The 3D image-based surgical planning method of claim 1, wherein the central area comprises a center and/or a near center of a corresponding vertebra body region and/or a pedicle region.

5. The 3D image-based surgical planning method of any one of claims 1 to 4, wherein the third image is obtained from the surgery site image based on a coordinate of the first baseline and a slope of the second baseline.

6. The 3D image-based surgical method of any one of claims 1 to 4, wherein the third image is obtained from the surgery site image, based on coordinates of the first baseline of the first image and the second baseline of the second image, wherein the second baseline commonly passes through the central area of each of the left and right pedicles in the second image.

7. The 3D image-based surgical planning method of claim 6, wherein the second baseline is set to be parallel with an arbitrary straight line connecting a midpoint between a center of a pedicle region at one side of the left and right pedicle regions, and a coordinate of a corner at one side of the third image adjacent to the center of the pedicle region with a midpoint between a center of a pedicle region at the other side of the left and right pedicle regions and a coordinate of a corner at the other side of the third image adjacent to the center of the pedicle region.

8. The 3D image-based surgical method of any one of claims 1 to 4, wherein

the object segmentation portion extracts the foramen region between the left and right third pedicle regions from the third image, and

the baseline setting portion sets, in the third image, the third baseline commonly passing through coordinates of a center or a near center of each of the third vertebra body region and the foramen region, and sets the distal setting coordinates on a point at which the third baseline crosses a boundary line of a vertebra body region most distanced apart from the foramen region or a position near to the point, or on a crossing point at which the third baseline and the baseline of the screw path cross each other outside the vertebra body.

9. The 3D image-based surgical planning method of claim 8, wherein the baseline setting portion sets the baseline of the screw path on which the screw path is arranged to pass through the distal setting coordinates.

10. The 3D image-based surgical planning method of any one of claims 1 to 4, wherein a screw start point with respect to a pedicle and a target point in a vertebra body are set on the first baseline, and a distance between the screw start point and the target point is set as a length of a screw used in surgery.

11. A three-dimensional (3D) image-based surgical planning system comprising:

one or more image acquisition devices obtaining one or more surgery site images having image elements in a plurality of directions with respect to a spinal surgery site;

an image processor configured to process images related to a surgery, including extraction of a first image in a first direction, a second image in a second direction different from the first direction, and a third image in a third direction different from the first direction and the second direction from the one or more surgery site images;

an object segmentation portion extracting a first vertebra body region and a first pedicle region corresponding to a vertebra body and a pedicle from the first image, a second vertebra body region and a second pedicle region in the second vertebra body region from the second image, and a third vertebra body region and a third pedicle region from the third image;

a baseline setting portion setting a first baseline passing through a first coordinate set in a central area of the first pedicle region and a second coordinate set in the first vertebra body region and a second baseline commonly passing through a third coordinate and fourth coordinates set in central areas of the left and right pedicle regions in the second image; and

a screw path setting portion setting a screw path passing through a central area of at least one of the left and right third pedicle regions and the third vertebra body region while setting proximal setting coordinates in the second pedicle, and setting, on a third baseline passing through a foramen region between the left and right third pedicle regions and a central area of the vertebra body, distal setting coordinates through which a baseline of the screw path passes.

12. The 3D image-based surgical planning system of claim 11, wherein the baseline setting portion arranges the first baseline to cross a midline passing through a region between the first pedicle region and the first vertebra body region, wherein the first baseline is set to pass through the central area of the first pedicle region or to be parallel by being apart from the central area by a distance arbitrarily set.

13. The 3D image-based surgical method of claim 11, wherein the image processor obtains the third image from the surgery site image, based on a coordinate of the first baseline and a slope of the second baseline.

14. The 3D image-based surgical system of claim 11, wherein the image processor obtains the third image from the surgery site image, based on coordinates of the first baseline of the first image and the second baseline of the second image, wherein the second baseline commonly passes through the central area of each of the left and right pedicles in the second image.

15. The 3D image-based surgical planning system of claim 14, wherein the second baseline is set to be parallel with an arbitrary straight line connecting a midpoint between a center of a pedicle region at one side of the left and right pedicle regions, and a coordinate of a corner at one side of the third image adjacent to the center of the pedicle region with a midpoint between a center of a pedicle region at the other side and a coordinate of a corner at the other side of the third image adjacent to the center of the pedicle region.

16. The 3D image-based surgical planning system of claim 11, wherein

the object segmentation portion extracts the foramen region between the left and right third pedicle regions from the third image, and

the path setting portion sets, in the third image, the third baseline commonly passing through coordinates of a center or a near center of each of the third vertebra body region and the foramen region, and sets the distal setting coordinates on a point at which the third baseline crosses a boundary line of a vertebra body region most distanced apart from the foramen region or a position near to the point, or on a crossing point at which the third baseline and the baseline of the screw path cross each other outside the vertebra body.

17. The 3D image-based surgical planning system of claim 16, wherein the path setting portion sets the baseline of the screw path on which the screw path is arranged to pass through the distal setting coordinates.

18. The 3D image-based surgical planning method of any one of claims 11 to 17, wherein a screw start point with respect to a pedicle and a target point in a vertebra body are set on the first baseline, and a distance between the screw start point and the target point is set as a length of a screw used in surgery.

**Amended claims under Art. 19.1 PCT**

1. A three-dimensional (3D) image-based surgical planning method comprising:

obtaining, via one or more image acquisition devices, one or more surgery site images comprising image information in a plurality of directions including a first direction, a second direction, and a third direction which are different from one another, with respect to a spinal surgery site of a patient;

extracting, from the surgery site image via an image processor, a first image in the first direction and a second image in the second direction;

extracting, via an object segmentation portion, a first vertebra body region and a first pedicle region from the first image and a second vertebra body region and left and right second pedicle regions in the second vertebra body region from the second image;

setting, via a baseline setting portion, a first baseline passing through a first coordinate set in a central area of the first pedicle region and a second coordinate set in the first vertebra body region and a second baseline passing through both of a third coordinate and fourth coordinates set in central areas of the left and right pedicle regions in the second image;

extracting, based on the first baseline and the second baseline via the image processor, a third image in the third direction from the surgery site image;

extracting, via the object segmentation portion, a third vertebra body region and left and right third pedicle regions from the third image; and

setting, via a path setting portion, a screw path passing through a central area of at least one of the left and right third pedicle regions and the third vertebra body region, while setting proximal setting coordinates in the second pedicle, and setting, on a third baseline passing through a foramen region between the left and right third pedicle regions and a central area of the vertebra body, distal setting coordinates through which a baseline of the screw path passes.

2.  The 3D image-based surgical planning method of claim 1, wherein the object segmentation portion extracts the vertebra body region and the pedicle region by applying a model generated by machine learning.

3.  The 3D image-based surgical planning method of claim 1, wherein the first baseline intersects a midline passing through a region between the first pedicle region and the first vertebra body region or crosses the midline by an arbitrary angle, wherein the first baseline is set to pass through the central area of the first pedicle region or to be parallel by being apart from the central area of the first pedicle region by a distance arbitrarily set.

4.  The 3D image-based surgical planning method of claim 1, wherein the central area comprises a center and/or a near center of a corresponding vertebra body region and/or a pedicle region.

5.  The 3D image-based surgical planning method of any one of claims 1 to 4, wherein the third image is obtained from the surgery site image based on a coordinate of the first baseline and a slope of the second baseline.

6.  The 3D image-based surgical planning method of any one of claims 1 to 4, wherein the third image is obtained from the surgery site image, based on coordinates of the first baseline of the first image and the second baseline of the second image, wherein the second baseline commonly passes through the central area of each of the left and right pedicles in the second image.

7.  The 3D image-based surgical planning method of claim 6, wherein the second baseline is set to be parallel with an arbitrary straight line connecting a midpoint between a center of a pedicle region at one side of the left and right pedicle regions, and a coordinate of a corner at one side of the third image adjacent to the center of the pedicle region with a midpoint between a center of a pedicle region at the other side of the left and right pedicle regions and a coordinate of a corner at the other side of the third image adjacent to the center of the pedicle region.

8.  The 3D image-based surgical planning method of any one of

    claims 1 to 4, wherein the object segmentation portion extracts the foramen region between the left and right third pedicle regions from the third image, and
    the baseline setting portion sets, in the third image, the third baseline commonly passing through coordinates of a center or a near center of each of the third vertebra body region and the foramen region, and sets the distal setting coordinates on a point at which the third baseline crosses a boundary line of a vertebra body region most distanced apart from the foramen region or a position near to the point, or on a crossing point at which the third baseline and the baseline of the screw path cross each other outside the vertebra body.

9.  The 3D image-based surgical planning method of claim 8, wherein the baseline setting portion sets the baseline of the screw path on which the screw path is arranged to pass through the distal setting coordinates.

10. The 3D image-based surgical planning method of any one of claims 1 to 4, wherein a screw start point with respect to a pedicle and a target point in a vertebra body are set on the first baseline, and a distance between the screw start point and the target point is set as a length of a screw used in surgery.

11. A three-dimensional (3D) image-based surgical planning system comprising:

one or more image acquisition devices obtaining one or more surgery site images having image elements in a plurality of directions with respect to a spinal surgery site;
an image processor configured to process images related to a surgery, including extraction of a first image in a first direction, a second image in a second direction different from the first direction, and a third image in a third direction different from the first direction and the second direction from the one or more surgery site images;
an object segmentation portion extracting a first vertebra body region and a first pedicle region corresponding to a vertebra body and a pedicle from the first image, a second vertebra body region and a second pedicle region in the second vertebra body region from the second image, and a third vertebra body region and a third pedicle region from the third image;
a baseline setting portion setting a first baseline passing through a first coordinate set in a central area of the first pedicle region and a second coordinate set in the first vertebra body region and a second baseline commonly passing through a third coordinate and fourth coordinates set in central areas of the left and right pedicle regions in the second image; and
a screw path setting portion setting a screw path passing through a central area of at least one of left and right third pedicles and the third vertebra body region while setting proximal setting coordinates in the second pedicle, and setting, on a third baseline passing through a foramen region between the left and right third pedicle regions and a central area of the vertebra body, distal setting coordinates through which a baseline of the screw path passes.

12. The 3D image-based surgical planning system of claim 11, wherein the baseline setting portion arranges the first baseline to cross a midline passing through a region between the first pedicle region and the first vertebra body region, wherein the first baseline is set to pass through the central area of the first pedicle region or to be parallel by being apart from the central area by a distance arbitrarily set.

13. The 3D image-based surgical planning system of claim 11, wherein the image processor obtains the third image from the surgery site image, based on a coordinate of the first baseline and a slope of the second baseline.

14. The 3D image-based surgical planning system of claim 11, wherein the image processor obtains the third image from the surgery site image, based on coordinates of the first baseline of the first image and the second baseline of the second image, wherein the second baseline commonly passes through the central area of each of the left and right pedicles in the second image.

15. The 3D image-based surgical planning system of claim 14, wherein the second baseline is set to be parallel with an arbitrary straight line connecting a midpoint between a center of a pedicle region at one side of the left and right pedicle regions, and a coordinate of a corner at one side of the third image adjacent to the center of the pedicle region with a midpoint between a center of a pedicle region at the other side and a coordinate of a corner at the other side of the third image adjacent to the center of the pedicle region.

16. The 3D image-based surgical planning system of claim 11, wherein

the object segmentation portion extracts the foramen region between the left and right third pedicle regions from the third image, and
the path setting portion sets, in the third image, the third baseline commonly passing through coordinates of a center or a near center of each of the third vertebra body region and the foramen region, and sets the distal setting coordinates on a point at which the third baseline crosses a boundary line of a vertebra body region most distanced apart from the foramen region or a position near to the point, or on a crossing point at which the third baseline and the baseline of the screw path cross each other outside the vertebra body.

17. The 3D image-based surgical planning system of claim 16, wherein the path setting portion sets the baseline of the screw path on which the screw path is arranged to pass through the distal setting coordinates.

**18.** The 3D image-based surgical planning method of any one of claims 11 to 17, wherein a screw start point with respect to a pedicle and a target point in a vertebra body are set on the first baseline, and a distance between the screw start point and the target point is set as a length of a screw used in surgery.

# FIG. 1

```
            START
              │
              ▼
     ┌──────────────────┐
     │ PREPARATORY WORK │
     └──────────────────┘
  ┌────────────────────────────┐
S1│ CAPTURE THREE-DIMENSIONAL   │
  │        (3D) IMAGE           │
  └────────────────────────────┘
              │
  ┌────────────────────────────┐
S2│    LOAD 3D IMAGE AND        │
  │       COORDINATES          │
  └────────────────────────────┘
              │
  ┌────────────────────────────┐
S3│        DRR IMAGE            │
  │  (ORTHOGONAL PROJECTION)    │
  └────────────────────────────┘
              │
  ┌────────────────────────────┐
S4│     AP/LL SEGMENTATION      │
  └────────────────────────────┘
              │
  ┌────────────────────────────┐
S5│      TARGET LABELLING       │
  └────────────────────────────┘
```

| Step | Process | Group |
|------|---------|-------|
| S6 | TARGET INPUT | (GENERATE LL POINT) |
| S7 | EXTRACT LL PARAMETER | |
| S8 | GENERATE AXIAL SLICE PATH (1) | |
| S9 | EXTRACT AP PARAMETER | (GENERATE AP POINT) |
| S10 | GENERATE AXIAL SLICE PATH (2) AND EXTRACT IMAGE | |
| S11 | EXTRACT AXIAL PARAMETER | |
| S12 | GENERATE AXIAL OPTIMAL PATH POINT | (GENERATE AXIAL POINT) |
| S13 | GENERATE OPTIMAL PATH | |
| S14 | LAST TARGET? — No / Yes | (OPTIMIZATION AND AUTOMATIZATION) |

END

EP 4 775 165 A1

# FIG. 2

(X)

(Z)

• 2D DRR IMAGE

C-am AP Cone

(Y)

(Z)

C-am LL Cone

Virtual
Space

$S_{LL}$
X-ray Source LL

(Y)

(Z)

(X)

• 3D IMAGE AND
COORDINATE DEFINITION

$S_{AP}$
X-ray Source AP

# FIG. 3

(Input)

LL Segmentation model

(Output)

(Pedicle)

(Vertebra body)

FIG. 4

(Input)                     AP
                         Segmentation
                           model

                                        Pedicle

                                        Vertebra
                                        body

(Input)

# FIG. 5

# FIG. 6

(Pedicle)

(Vertebra body)

# FIG. 7

(Pedicle)

(Vertebra body)

# FIG. 8

(Axial slice path)          (Annotation & leanning)

# FIG. 9

Target(L1)
Target(L2)
Target(L3)
Target(L4)
Target(L5)

(AP)

Target(L1)
Target(L2)
Target(L3)
Target(L4)
Target(L5)

(LL)

FIG. 10

(LL – L1)                    (AP – L1)

FIG. 11

FIG. 12

FIG. 13

# FIG. 14A

# FIG. 14B

# FIG. 15A

FIG. 15B

FIG. 16

# FIG. 17

# FIG. 18

(D)

(d)

1ST IMAGE

2ND IMAGE

FIG. 19

(A)

(B)

EP 4 775 165 A1

# FIG. 20A

(A)

(B)

(C)

(D)

# FIG. 20B

# FIG. 21A

(A)

(B)

EP 4 775 165 A1

FIG. 21B

(A)

(B)

# FIG. 22A

(A)

(B)

EP 4 775 165 A1

FIG. 22B

(A)

(B)

# FIG. 23

## INTERNATIONAL SEARCH REPORT

| International application No. |
|---|
| **PCT/KR2024/012185** |

### A. CLASSIFICATION OF SUBJECT MATTER

**A61B 34/10**(2016.01)i; **A61B 17/70**(2006.01)i; **G06T 7/11**(2017.01)i; **G06T 7/30**(2017.01)i

According to International Patent Classification (IPC) or to both national classification and IPC

### B. FIELDS SEARCHED

Minimum documentation searched (classification system followed by classification symbols)

A61B 34/10(2016.01); A61B 5/00(2006.01); A61B 90/00(2016.01); G06T 11/00(2006.01); G06T 7/00(2006.01); G06T 7/33(2017.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 영상 기반 3D 수술 계획(video-based 3D surgical planning), 척추 스크류(spinal screw), 방향 영상 추출(directional video extraction), 해부학적 영역 추출(anatomical region extraction), 기준선(reference line), 스크류 패스 설정(screw path setting)

### C. DOCUMENTS CONSIDERED TO BE RELEVANT

| Category* | Citation of document, with indication, where appropriate, of the relevant passages | Relevant to claim No. |
|---|---|---|
| A | KR 10-2021-0123912 A (CUREXO INC.) 14 October 2021 (2021-10-14)<br>See claims 1-4 and 14. | 1-18 |
| A | KR 10-2020-0109489 A (CUREXO INC.) 23 September 2020 (2020-09-23)<br>See entire document. | 1-18 |
| A | US 2017-0323443 A1 (INDIAN INSTITUTE OF TECHNOLOGY, BOMBAY) 09 November 2017 (2017-11-09)<br>See entire document. | 1-18 |
| A | KR 10-2015-0026354 A (SAMSUNG ELECTRONICS CO., LTD.) 11 March 2015 (2015-03-11)<br>See entire document. | 1-18 |

☑ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **21 November 2024** | **21 November 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

| International application No. |
| --- |
| **PCT/KR2024/012185** |

**C. DOCUMENTS CONSIDERED TO BE RELEVANT**

| Category* | Citation of document, with indication, where appropriate, of the relevant passages | Relevant to claim No. |
| --- | --- | --- |
| A | USHAKIRAN et al. Feature-based registration framework for pedicle screw trajectory registration between multimodal images. IEEE Access. 2023 (online publication date: 15 June 2023), vol. 11, pp. 59816-59826.<br>    See entire document. | 1-18 |

Form PCT/ISA/210 (second sheet) (July 2022)

**INTERNATIONAL SEARCH REPORT**
Information on patent family members

International application No.

**PCT/KR2024/012185**

| Patent document cited in search report | | | Publication date (day/month/year) | Patent family member(s) | | | Publication date (day/month/year) |
|---|---|---|---|---|---|---|---|
| KR | 10-2021-0123912 | A | 14 October 2021 | CN | 115426967 | A | 02 December 2022 |
| | | | | EP | 4134034 | A1 | 15 February 2023 |
| | | | | JP | 2023-520602 | A | 17 May 2023 |
| | | | | KR | 10-2394901 | B1 | 09 May 2022 |
| | | | | US | 11666458 | B2 | 06 June 2023 |
| | | | | US | 2023-0127917 | A1 | 27 April 2023 |
| | | | | WO | 2021-206372 | A1 | 14 October 2021 |
| KR | 10-2020-0109489 | A | 23 September 2020 | CN | 113556977 | A | 26 October 2021 |
| | | | | CN | 113556977 | B | 05 July 2024 |
| | | | | EP | 3939509 | A2 | 19 January 2022 |
| | | | | JP | 2022-523587 | A | 25 April 2022 |
| | | | | JP | 7213413 | B2 | 27 January 2023 |
| | | | | KR | 10-2203544 | B1 | 18 January 2021 |
| | | | | US | 11337763 | B2 | 24 May 2022 |
| | | | | US | 2022-0039874 | A1 | 10 February 2022 |
| | | | | WO | 2020-185048 | A2 | 17 September 2020 |
| | | | | WO | 2020-185048 | A3 | 29 October 2020 |
| US | 2017-0323443 | A1 | 09 November 2017 | US | 10217217 | B2 | 26 February 2019 |
| | | | | WO | 2016-116946 | A2 | 28 July 2016 |
| | | | | WO | 2016-116946 | A3 | 15 September 2016 |
| | | | | WO | 2016-116946 | A9 | 13 October 2016 |
| KR | 10-2015-0026354 | A | 11 March 2015 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8335553 B
- CN 107157579
- US 7787932 B2
- KR 101264198 B1
- US 0659599 B2
- US 6226548 B1
- US 20070270866 A1
- US 20050192575 A1